# EUROPÄISCHE PATENTSCHRIFT

(11) **EP 2 290 354 B1**
(45) Veröffentlichungstag und Bekanntmachung des Hinweises auf die Patenterteilung: **24.07.2019**
(21) Anmeldenummer: 09168536.2
(22) Anmeldetag: 25.08.2009
(51) Int. Cl.: G01N 21/11, G01N 21/85, B01L 3/00, F04B 43/12, F04B 43/08, G01N 35/10, G01N 21/03, G01N 21/05, G01N 21/78, G01N 21/84, G01N 33/18, G01N 1/40

(54) **Prozess-Analysegerät**
Process analyser
Appareil d'analyse de processus

(43) Veröffentlichungstag der Anmeldung: 02.03.2011
(60) Teilanmeldung: 19181326.0
(73) Patentinhaber: Hach Lange GmbH, 14163 Berlin (DE)
(72) Erfinder: Lundgreen, Dr., Ulrich, 33330 Gütersloh (DE); Farjam, Dr., Aria, 40223 Düsseldorf (DE)
(74) Vertreter: Patentanwälte ter Smitten Eberlein-Van Hoof Rütten Partnerschaftsgesellschaft mbB

(56) Entgegenhaltungen:
- EP-A- 1 870 033
- DE-A1- 10 126 054
- DE-A1- 10 214 781
- DE-A1- 10 227 032
- US-A- 5 646 863
- US-A1- 2007 144 277
- US-A1- 2008 026 373
- US-B1- 6 852 284

## Beschreibung

Die Erfindung bezieht sich auf ein Prozess-Analysegerät, wie es beispielsweise in der Wasser-Analytik in Form einer Tauchsonde, einer Rohreinbausonde oder eines Labor-Analysegerätes eingesetzt wird.

Prozess-Analysegeräte führen quasi-kontinuierlich Analysen zur quantitativen Bestimmung eines Analyts in Wasser durch, und weisen hierfür einen oder mehrere Vorratstanks zur Bevorratung von Flüssigkeiten, z.B. Trägerflüssigkeiten und/oder Reagenzien auf, die für die Durchführung der Wasser-Analyse benötigt werden. Für den Transport der Flüssigkeiten in dem Analysegerät sind Pumpen und Flüssigkeitsleitungen vorgesehen.

Bei Erschöpfung eines Vorrats in einem Vorratstank muss der betreffende Vorratstank wieder aufgefüllt werden. Im praktischen Betrieb treten ferner häufig Störungen auf, die insbesondere durch Ablagerungen und Auskristallisationen in den Flüssigkeitsleitungen oder auch in den flüssigkeitsführenden Teilen der Pumpen verursacht werden. Diese Störungen, sowie normale Abnutzung von Verschleißteilen wie z.B. Schlauchpumpen-Schläuchen, führen dazu, dass zusätzlich zu der regelmäßigen Auffüllung der Vorratstanks auch Wartungen und Reparaturen fällig werden, z.B. der Austausch von Schläuchen.

Aus DE 101 26054 A1 ist ein Analysegerät bekannt, das aus einem Basismodul und einem austauschbaren Kartuschenmodul besteht. Das Basismodul weist eine Analyseeinheit auf, so dass zwischen dem Basismodul und dem Kartuschenmodul eine Vielzahl von Flüssigkeitsleitungs-Schnittstellen vorhanden ist. Alternativ kann die Analyseeinheit auch vollständig in dem Kartuschenmodul angeordnet sein, so dass in diesem Fall eine Vielzahl elektrischer Schnittstellen zwischen dem Basismodul und dem Kartuschenmodul erforderlich ist.

Aufgabe der Erfindung ist es demgegenüber, ein Prozess-Analysegerät mit hoher Betriebssicherheit und geringem Wartungsaufwand zu schaffen.

Diese Aufgabe wird erfindungsgemäß durch ein Prozess-Analysegerät zur Bestimmung eines Analyts in Wasser, mit den Merkmalen des Patentanspruches 1 gelöst.

Das erfindungsgemäße Prozess-Analysegerät ist modular aufgebaut, und besteht aus einem ständig verwendeten Basismodul und einem austauschbaren Kartuschenmodul.

Das Basismodul weist einen Pumpen-Antriebsmotor und einen Analyt-Sensor ohne flüssigkeitsführende Messtrecke auf. Das Basismodul weist keinerlei flüssige Fluidik auf, also keine flüssigkeitsführenden Leitungen, Ventile, Messstrecken oder Pumpmimiken.

Das Kartuschenmodul dagegen weist mindestens einen Flüssigkeits-Vorratstank,
eine Probengewinnungs-Vorrichtung,
eine antriebslose Pumpmimik zum Pumpen der Flüssigkeit aus dem Vorratstank, und eine Messstrecke für den Analytsensor auf. Die gesamte flüssige Fluidik ist also in dem Kartuschenmodul angeordnet. Unter Pumpmimik ist der fluidische Transportmechanismus zu verstehen, d.h. der Teil der Pumpe, der in direktem Kontakt zur Flüssigkeit steht und den Transport innerhalb des fluidischen Systems bewirkt.

Bei in das Basismodul eingesetztem Kartuschenmodul wird die Pumpmimik über das Kupplungsteil durch den Pumpenantrieb angetrieben, und ist die flüssigkeitsführende Messstrecke dem Analyt-Sensor derart zugeordnet, dass dieser Messungen von in der Flüssigkeit enthaltenen Analyten durchführen kann.

Bei Erschöpfung der Flüssigkeit In dem Vorratstank, beispielsweise einer Erschöpfung von Trägerflüssigkeit oder Reagenz, wird das Kartuschenmodul mit der gesamten Fluidik ausgetauscht. Der Austausch der gesamten Fluidik erfolgt also regelmäßig beziehungsweise verbrauchsgesteuert. Eine separate Wartung der Fluidik des Analysegerätes über das Auffüllen des Flüssigkeits-Vorratstanks hinaus entfällt. Der Wartungsaufwand ist dadurch erheblich reduziert. Durch das regelmäßige beziehungsweise verbrauchsgesteuerte Austauschen der gesamten Fluidik werden Störungen, die durch Ablagerungen in der Fluidik oder Verschleiß fluidischer Komponenten, z.B. Pumpenschläuche, verursacht werden können, auf ein Minimum reduziert.

Vorzugsweise ist die Pumpmimik als Peristaltik ausgebildet, und besonders bevorzugt als pneumatische peristaltische Membranpumpe. Eine peristaltische Pumpmimik ist konstruktiv sehr einfach, und kann beispielsweise von einem flexiblen Abschnitt der Flüssigkeitsleitung gebildet werden, der von dem Kopplungsteil in einer Längsrichtung fortlaufend verformt wird.

Das Kopplungsteil kann beispielsweise in Form von drei in Längsrichtung hintereinander angeordneten Quetschkolben ausgebildet sein, die in einer Längsrichtung nacheinander den Flüssigkeitsleitungs-Abschnitt flüssigkeitsdicht abquetschen, und auf diese Weise eine peristaltische Pumpbewegung erzeugen. Eine besonders bevorzugte Ausführungsform ist die pneumatische Peristaltikpumpe, bei der die mechanische Kopplung durch Anlegen von Über- bzw. Unterdruck auf eine mehrstufige Membranpumpe erzeugt wird. Der Pumpenantrieb kann beispielsweise elektromechanisch oder pneumatisch ausgebildet sein.

Ein Lufteintrag in einer Flüssigkeitsleitung stellt für eine peristaltische Pumpmimik kein Problem dar, da diese gegen Luft ansaugen kann. Ferner kann bei Verwendung einer peristaltischen Pumpmimik auf Ventile, insbesondere auf anfällige Rückschlag-Ventile verzichtet werden. Die peristaltische Pumpmimik bietet ferner die Möglichkeit, mit einer einzigen peristaltischen Pumpmimik mehrere Leitungen synchron, das heißt, mehrere Flüssigkeiten gleichzeitig, pumpen zu können. Schließlich ist die peristaltische Pumpmimik eine ausgereifte, preiswerte und äußerst zuverlässige Pumptechnik.

Gemäß einer bevorzugten Ausgestaltung weist das Basismodul mehrere voneinander unabhängige Pumpenantriebe auf, die mehreren voneinander unabhängigen Pumpmimiken in dem Kartuschenmodul zugeordnet sind. Zwar können grundsätzlich auch mit einem einzigen Pumpenantrieb mehrere Pumpmimiken angetrieben werden. Vorteilhaft ist jedoch, jeder Pumpmimik in dem Kartuschenmodul basismodulseitig einen eigenen Pumpenantrieb zuzuordnen. Auf diese Weise können mehrere Flüssigkeitsströme unabhängig voneinander gesteuert werden.

Vorzugsweise weist das Kartuschenmodul mehrere Vorratstanks auf. Als Vorratstank ist vorliegend ein Tank für ein Reagenz, eine Trägerflüssigkeit, eine Reinigungsflüssigkeit oder auch für Abfallflüssigkeit zu verstehen.

Gemäß einer bevorzugten Ausgestaltung weist der Analyt-Sensor basismodulseitig ein Fotometer zur fotometrischen Bestimmung eines Analyts in einer Wasserprobe auf. Die Wasserprobe kann beispielsweise dialytisch beispielsweise aus Abwasser gewonnen werden. Hierzu weist die Probengewinnungs-Vorrichtung des Kartuschenmoduls in einer bevorzugten Ausführungsform eine Dialysemembran auf. Besonders bevorzugt ist das vorliegende Analysegerät als flüssigkeitsdichte Abwasser-Tauchsonde ausgebildet.

Im Folgenden wird die Erfindung anhand der Zeichnungen näher erläutert.

Es zeigen:
- Figur 1: eine schematische Darstellung eines als Tauchsonde ausgebildeten Analysegerätes,
- Figur 2: eine erste Ausführungsform eines basisseitigen pneumatischen Pumpenantriebs und einer damit zusammenwirkenden kartuschenseitigen Pumpmimik, und
- Figur 3: eine zweite Ausführungsform eines basisseitigen elektromechanischen Pumpenantriebs und einer damit zusammenwirkenden kartuschenseitigen Pumpmimik.

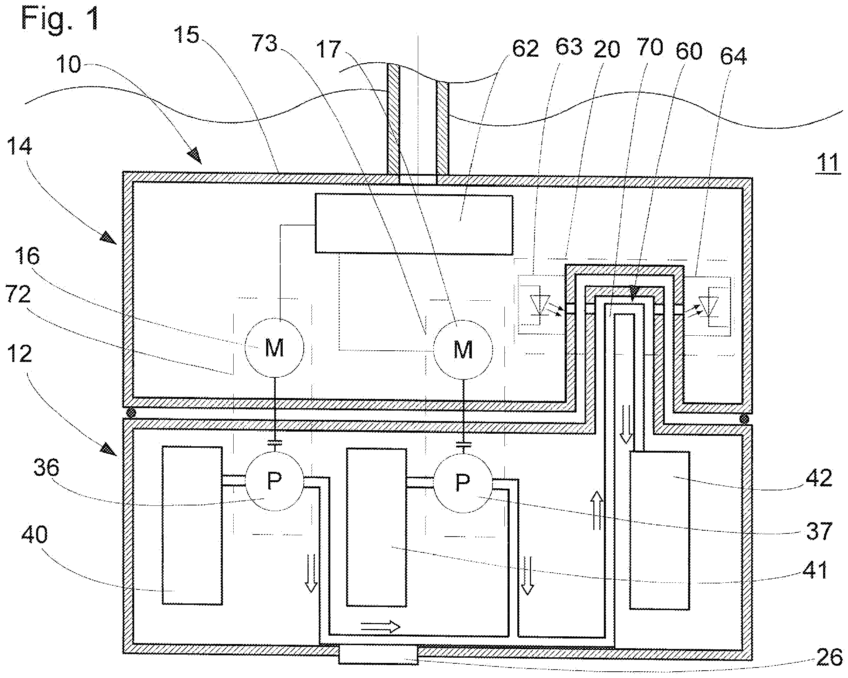

In der Figur 1 ist schematisch ein Prozess-Analysegerät 10 dargestellt, das als Tauchsonde ausgebildet ist, und der Analyse von Wasser 11 zur Regelung eines Klärprozesses dient. Das Analysegerät 10 ist zweiteilig und modular aufgebaut, und besteht aus einem flüssigkeitsdichten Basismodul 14 und einem austauschbaren flüssigkeitsdichten Kartuschenmodul 12. Das Basismodul 14 weist ein Gehäuse 15 auf, in das bzw. an das das Kartuschenmodul 12 ein- bzw. angesteckt ist.

Das Basismodul 14 weist einen als Fotometer ausgebildeten Analyt-Sensor 20 ohne Fluidik-Messtrecke 60 auf, die in dem Kartuschenmodul 12 vorgesehen ist. Der Analyt-Sensor 20 weist basismodulseitig eine Lichtquelle 63 und einen Detektor 64 in Form einer Photozelle auf.

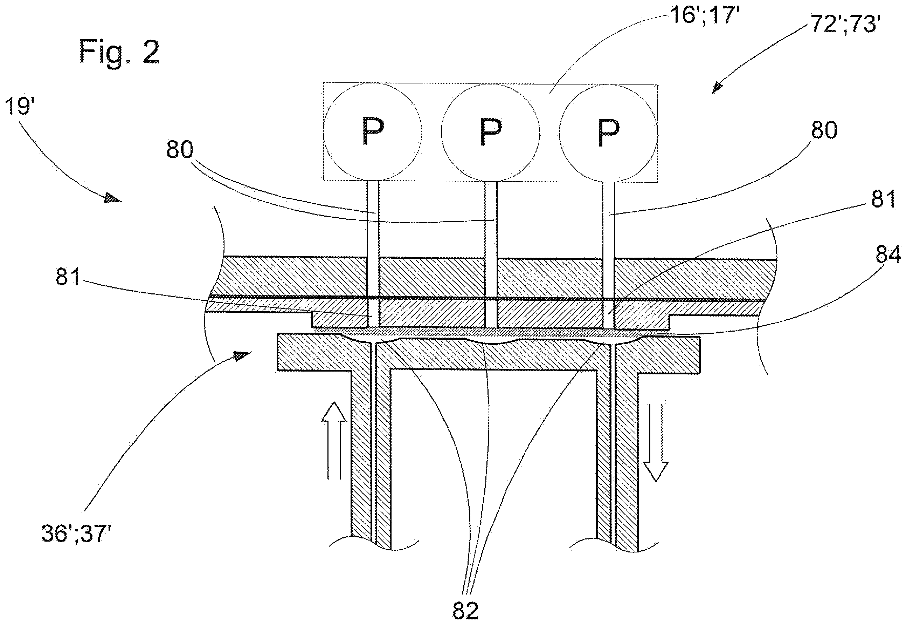

Wie in Figur 2 dargestellt ist, kann die Pumpe gemäß einer ersten Ausführungsform pneumatisch ausgebildet sein, wobei der Pumpenantrieb 16' als Luftpumpe ausgebildet ist, die drei Luftkanäle 80 sukzessive mit Überdruck und ggfs. auch mit Unterdruck beaufschlagt, die mit entsprechenden Luftkanälen 81 des Kartuschenmoduls 12 gekoppelt sind. Die kartuschenmodulseitigen Luftkanäle 81 sind durch eine Pumpmembran 84 verschlossen, unter der drei Pumpkammern 82 gebildet sind.

Wird in dem Luftkanal 80 durch den Pumpenantrieb 16' Überdruck angelegt, dehnt sich die Pumpmembran 84 in die darunter liegende Pumpkammer 82 aus und verdrängt die darin befindliche Flüssigkeit. Wird Unterdruck angelegt, legt sich die Pumpmembran 84 glatt an die Oberfläche der Luftseite an, und die Pumpkammer 82 füllt sich wieder mit Flüssigkeit. Durch wechselndes Anlegen von Über- und Unterdruck in der entsprechenden Reihenfolge der drei aufeinanderfolgenden Pumpkammern 82 wird eine peristalitsche Fortbewegung der Flüssigkeit durch alle drei Pumpkammern 82 und im nachfolgenden fluidischen System erreicht.

In dem Pumpenantrieb 16' sind drei nicht dargestellte Ventile vorgesehen, die entweder Überdruck oder Unterdruck in den jeweiligen Luftkanal 80 durchschalten. Die in den Figuren nicht dargestellten Ventile sind demnach die Kopplung zwischen der Druckluftpumpe und der Pumpmimik 36'.

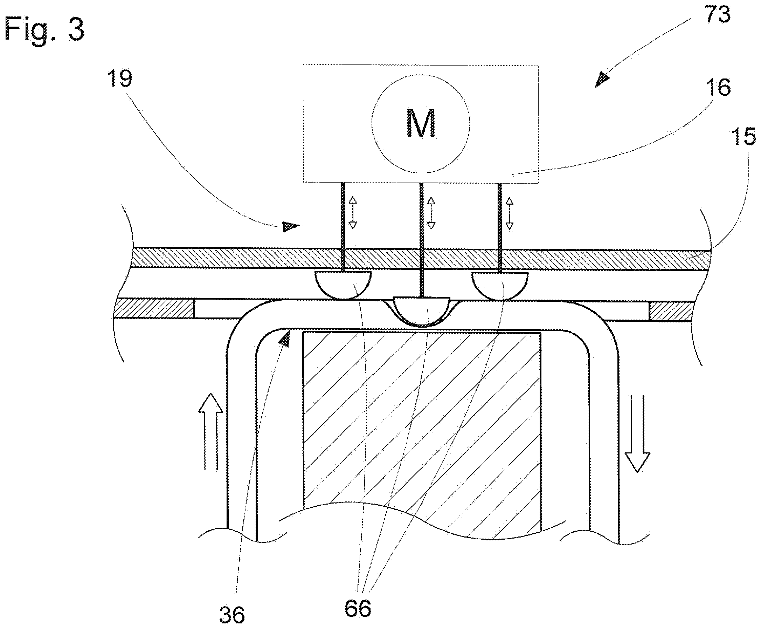

Das Basismodul 14 weist als Pumpenantrieb alternativ einen Antriebsmotor 16,17 mit einem Kopplungsteil 19 auf, das in der Figur 3 dargestellt ist. Das Kopplungsteil 19 besteht aus drei peristaltischen Quetschkolben 66, die eine peristaltische Bewegung in einer als flexibler Leitungsabschnitt ausgebildeten Pumpmimik 36 erzeugen können.

Zur Steuerung des Analysegerätes 10 weist das Basismodul 14 ferner eine Steuerung 62 auf.

Das Kartuschenmodul 12 weist drei Vorratstanks 40,41,42 auf, in denen eine Trägerflüssigkeit, ein Reagenz sowie Abfallflüssigkeit gelagert sind. Der Trägerflüssigkeits-Tank 40 und der Reagenz-Tank 41 sind jeweils über eine Flüssigkeitsleitung an eine eigene peristaltische Pumpmimik 36,37 angeschlossen, durch die die Trägerflüssigkeit beziehungsweise das Reagenz fein dosiert gepumpt werden kann. Der dritte Vorratstank 42 ist ein Tank für Abfallflüssigkeit, in den die von dem Analysator 20 kommende Abfall-Flüssigkeit fließt.

Die peristaltischen Pumpmimiken 36,37 der zweiten Ausführungsform sind zusammen mit den zugeordneten Kopplungsteilen 19 derart ausgelegt, dass ein unerwünschter Rückfluss oder Leckfluss von Trägerflüssigkeit oder Reagenz nicht auftreten kann. Die peristaltischen Pumpmimiken 36,37 sind kartuschenmodulseitig im Wesentlichen durch flexible Abschnitte der betreffenden Flüssigkeitsleitung gebildet, die durch die betreffenden Kopplungsteile 19 der zugeordneten Antriebsmotoren 16,17 angetrieben werden. Die Fluidik- Pumpe 72,73 der zweiten Ausführungsform wird also von dem Antriebsmotor 16, dem Kopplungsteil 19 und der Pumpmimik 36 gebildet.

Ferner weist das Kartuschenmodul 12 eine ionenselektive Dialysemembran 26 auf, durch die Analyt-Moleküle aus dem Wasser 11 in die innenseitig vorbei fließende Trägerflüssigkeit wandern können, Partikel, Makromoleküle und Mikroorganismen aber vom Durchtritt abgehalten werden. Die Membran 26 weist bevorzugt eine Porengröße von weniger als 2 µm auf.

Schließlich weist das Kartuschenmodul 12 die Fluidik-Messtrecke 60 auf, die von einem geraden Leitungsabschnitt 70 gebildet wird, dessen Längsenden transparente, lichtdurchlässige Fenster für die Fotometrie durch den Analyt-Sensor 20 aufweisen.

Wenn das Kartuschenmodul 12 an das Basismodul 14 montiert ist, ist die Messtrecke 60 genau ausgerichtet mit den basismodulseitigen Teilen des Analyt-Sensors 20. Ferner sind die Kopplungsteile 19 der Pumpen 72, 73 in peristaltischem Eingriff mit den kartuschenmodulseitigen Pumpmimiken 36.

Im Analysebetrieb wird aus dem Trägerflüssigkeits-Vorratstank 40 durch die zugeordnete Pumpe 72 Trägerflüssigkeit zu der Dialysemembran 26 gepumpt, durch die Analyt-Ionen aus dem Wasser 11 in die Trägerflüssigkeit wandern. Anschließend wird der mit den Analyt-Ionen angereicherten Trägerflüssigkeit ein Reagenz aus dem Reagenz-Vorratstank 41 durch die zugeordnete Pumpe 73 beigefügt, durch das das zu untersuchende Analyt in der Trägerflüssigkeit entsprechend seiner Konzentration eingefärbt wird. Die eingefärbte Probe wird zu der Messstrecke 60 gepumpt und dort durch den Analyt-Sensor 20 fotometrisch untersucht. Anschließend wird die Probe in den Abfall-Vorratstank 42 gepumpt.

Bei Erschöpfung von Reagenz oder Trägerflüssigkeit in den entsprechenden Vorratstanks 40,41, bei drohender Überfüllung des Abfall-Vorratstanks 42 oder bei einer Fehlfunktion, die möglicherweise auf eine der Komponenten des Kartuschenmoduls 12 zurückgeht, wird das Kartuschenmodul 12 ausgetauscht. Hierbei wird eine nicht dargestellte Verriegelung entriegelt und das Kartuschenmodul 12 axial nach unten von dem Basismodul-Gehäuse 15 abgezogen. Hierbei wird das Pumpen-Kopplungsteil 19 von der Pumpmimik 36 gelöst und wird die Messtrecke 60 aus dem Analyt-Sensor 20 entfernt. Anschließend kann ein neues Kartuschenmodul 12 an das Gehäuse 15 angesetzt werden, wobei die Pumpen-Kopplungsteile 19 wieder mit den Pumpmimiken 36 verbunden werden und die Messstrecke 60 in den Analysator 20 eingeführt wird.

## Patentansprüche

1. Prozess-Analysegerät (10) zur Bestimmung eines Analyts in Wasser, mit einem Basismodul (14;112) und einem austauschbaren Kartuschenmodul (12), wobei
das Basismodul (14) keine flüssige Fluidik aufweist, wobei das Basismodul (14) einen Pumpenantrieb (16,16') und einen Analyt-Sensor (20) ohne Fluidik-Messstrecke (60) aufweist, und
das Kartuschenmodul (12) die gesamte flüssige Fluidik aufweist, wobei das Kartuschenmodul (12) einen Flüssigkeits-Vorratstank (40,41), eine Probengewinnungs-Vorrichtung, eine antriebslose Pumpmimik (36,36'), die die Flüssigkeit aus dem Vorratstank (40,41) pumpt, und die Fluidik-Messstrecke (60) für den Analyt-Sensor (20) aufweist,
wobei bei in das Basismodul (14) eingesetztem Kartuschenmodul (12) die Pumpmimik (36,36') mit dem Pumpenantrieb (16,16') verbunden und durch den Pumpenantrieb (16,16') angetrieben wird, und die Fluidik-Messstrecke (60) mit dem Analyt-Sensor (20) verbunden ist.

2. Prozess-Analysegerät (10) nach Anspruch 1, wobei die Pumpmimik (36, 36') als Peristaltik ausgebildet ist.

3. Prozess-Analysegerät (10) nach Anspruch 1 oder 2, wobei die Pumpmimik (36,36') als peristaltische Membranpumpe ausgebildet ist, und besonders bevorzugt pneumatisch angetrieben wird.

4. Prozess-Analysegerät (10) nach einem der vorangegangenen Ansprüche, wobei das Kartuschenmodul (12) mehrere Vorratstanks (40,41) aufweist.

5. Prozess-Analysegerät (10) nach einem der vorangegangenen Ansprüche, wobei das Basismodul (14) mehrere voneinander unabhängige Pumpenantriebe (16,17,16',17') aufweist, die mehreren voneinander unabhängigen Pumpmimiken (36,37,36',37') in dem Kartuschenmodul (12) zugeordnet sind.

6. Prozess-Analysegerät (10) nach einem der vorangegangenen Ansprüche, wobei der Analyt-Sensor (20) einen optischen Detektor (64) aufweist, der die optische Bestimmung eines Analyts in der Fluidik-Messstrecke (60) erlaubt.

7. Prozess-Analysegerät (10) nach Anspruch 6, wobei der optische Detektor (64) die Absorption, die Fluoreszenz oder die Lumineszenz der Probe ermittelt.

8. Prozess-Analysegerät (10) nach einem der vorangegangenen Ansprüche, wobei das Analysegerät (10) als Abwasser-Tauchsonde ausgebildet ist.

9. Prozess-Analysegerät (10) nach einem der vorangegangenen Ansprüche, wobei die Probengewinnungs-Vorrichtung eine Dialyse-Membran (26) zur Gewinnung einer Probe aufweist.

10. Prozess-Analysegerät (10) nach einem der vorangegangenen Ansprüche, wobei das Kartuschenmodul (12) flüssigkeitsdicht gekapselt ist, so dass die darin enthaltenen Flüssigkeiten nicht nach Außen treten können.

## Claims

1. A process analysis unit (10) for determining an analyte in water, comprising a base module (14; 112) and an exchangeable cartridge module (12), wherein
the base module does not comprise liquid fluidics, wherein the base module (14) comprises a pump drive (16, 16') and an analyte sensor (20) without a fluidic measuring section (60), and
the cartridge module (12) comprises the entire liquid fluidics, wherein
the cartridge module (12) comprises a liquid reservoir tank (40, 41), a sample taking device, a drive-less pump mechanism (36, 36') pumping the liquid from the reservoir tank (40, 41), and the fluidic measuring section (60) for the analyte sensor (20),
wherein, when the cartridge module (12) is inserted in the base module (14), the pump mechanism (36, 36') is connected to the pump drive (16, 16') and is driven by the pump drive (16, 16'), and the fluidic measuring section (60) is connected to the analyte sensor (20).

2. The process analysis unit (10) of claim 1, wherein the pump mechanism (36, 36') is designed as a peristaltic device.

3. The process analysis unit (10) of claim 1 or 2, wherein the pump mechanism (36, 36') is configured as a peristaltic membrane pump and, particularly preferred, is driven pneumatically.

4. The process analysis unit (10) of one of the preceding claims, wherein the cartridge module (12) comprises a plurality of reservoir tanks (40, 41).

5. The process analysis unit (10) of one of the preceding claims, wherein the base module (14) comprises a plurality of independent pump drives (16, 17, 16', 17') associated to a plurality of independent pump mechanisms (36, 37, 36', 37') in the cartridge module (12).

6. The process analysis unit (10) of one of the preceding claims, wherein the analyte sensor (20) comprises an optical detector (64) allowing the optical determination of an analyte in the fluidic measuring section (60).

7. The process analysis unit (10) of claim 6, wherein the optical detector (64) determines the absorption, the fluorescence or the luminescence of the sample.

8. The process analysis unit (10) of one of the preceding claims, wherein the analysis unit (10) is designed as a waste-water immersion probe.

9. The process analysis unit (10) of one of the preceding claims, wherein the sample taking device comprises a dialysis membrane (26) for taking a sample.

10. The process analysis unit (10) of one of the preceding claims, wherein the cartridge module (12) is encapsulated in a liquid-tight manner so that the liquids contained therein cannot leak outwardly.

## Revendications

1. Appareil d'analyse de processus (10) destiné à la détermination d'un analyte dans l'eau, avec un module de base (14; 112) et un module de cartouche (12) échangeable, dans lequel
le module de base (12) ne comporte une fluidique fluide, le module de base (14) comprenant un entrainement de pompe (16, 16') et un capteur d'analyte (20) sans section de mesure fluidique (60), et
le module de cartouche (12) comprend toute la fluidique liquide, le module de cartouche (12) comprenant un réservoir de stockage de liquide (40, 41), un dispositif de prélèvement d'échantillon, un système de pompage (36, 36') non-entrainé qui pompe le liquide du réservoir de stockage de liquide (40, 41) et comprend la section de mesure fluidique (60) pour le capteur d'analyte (20),
dans lequel, avec le module de cartouche (12) placé dans le module de base (14), le système de pompage (36, 36') est relié avec l'entrainement de pompe (16, 16') et est entrainé par ledit entrainement de pompe (16, 16'), et la section de mesure fluidique (60) est reliée avec le capteur d'analyte (20).

2. Appareil d'analyse de processus (10) selon la revendication 1, dans lequel le système de pompage (36, 36') est conçu comme un système péristaltique.

3. Appareil d'analyse de processus (10) selon la revendication 1 ou 2, dans lequel le système de pompage (36, 36') est conçu comme une pompe péristaltique à membrane et, de manière particulièrement préférée, est entrainé pneumatiquement.

4. Appareil d'analyse de processus (10) selon l'une quelconque des revendications précédentes, dans lequel le module de cartouche (12) comprend plusieurs réservoirs de stockage (40, 41).

5. Appareil d'analyse de processus (10) selon l'une quelconque des revendications précédentes, dans lequel le module de base (14) comprend plusieurs entrainements de pompe (16, 17, 16', 17') indépendants l'un de l'autre, qui sont associés à plusieurs systèmes de pompage (36, 37, 36', 37') dans le module de cartouche, indépendants l'un de l'autre.

6. Appareil d'analyse de processus (10) selon l'une quelconque des revendications précédentes, dans lequel le capteur d'analyte (20) comprend un détecteur (64) optique permettant la détermination optique d'un analyte dans section de mesure fluidique (60).

7. Appareil d'analyse de processus (10) selon la revendication 6, dans lequel ledit détecteur (64) optique détermine l'absorption, la fluorescence ou la luminescence dudit échantillon.

8. Appareil d'analyse de processus (10) selon l'une quelconque des revendications précédentes, dans lequel ledit appareil d'analyse (10) est conçu comme une cloche d'immersion dans eaux usées.

9. Appareil d'analyse de processus (10) selon l'une quelconque des revendications précédentes, dans lequel le dispositif d'obtention d'échantillons comprend une membrane de dialyse (26) pour l'obtention d'un échantillon.

10. Appareil d'analyse de processus (10) selon l'une quelconque des revendications précédentes, dans lequel le module de cartouche (12) est encapsulé de manière étanche à liquide de sorte que les liquides contenus là-dedans ne puissent pas sortir vers l'extérieur.
